(19) Europäisches Patentamt
European Patent Office
Office européen des brevets

(11) EP 0 486 964 B1

(12) **EUROPÄISCHE PATENTSCHRIFT**

(45) Veröffentlichungstag und Bekanntmachung des Hinweises auf die Patenterteilung:
**01.05.1996 Patentblatt 1996/18**

(51) Int. Cl.$^6$: **C07C 57/30**, A61K 31/19, C07C 59/08, C07C 59/64

(21) Anmeldenummer: **91119524.6**

(22) Anmeldetag: **15.11.1991**

(54) **Komplexe, enthaltend S(+)-Phenyl-alkansäuren und alpha-Hydroxy-alkansäuren**
S(+)-Phenyl-alkanoic acids and alpha-hydroxy-alkanoic acids containing complexes
Complexes contenant acides S(+)-phénylalkanoiques et acides alpha-hydroxyalkanoiques

(84) Benannte Vertragsstaaten:
**AT BE CH DE DK ES FR GB IT LI LU NL SE**

(30) Priorität: **15.11.1990 DE 4036459**

(43) Veröffentlichungstag der Anmeldung:
**27.05.1992 Patentblatt 1992/22**

(73) Patentinhaber: **MEDICE**
**Chem.-Pharm. Fabrik**
**Pütter GmbH & Co. KG**
**D-58638 Iserlohn (DE)**

(72) Erfinder: **Paradies, Henrich Hasko, Prof. Dr. Dr.**
**W-5860 Iserlohn (DE)**

(74) Vertreter: **Reinhard - Skuhra - Weise & Partner**
**Postfach 44 01 51**
**D-80750 München (DE)**

(56) Entgegenhaltungen:
**DE-A- 3 836 863**

Bemerkungen:
Die Akte enthält technische Angaben, die nach dem Eingang der Anmeldung eingereicht wurden und die nicht in dieser Patentschrift enthalten sind.



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.11.3/3.4

## Beschreibung

Die vorliegende Erfindung betrifft wasserstoffbrücken-gebundene Komplexe mit einer Stöchiometrie von 1:1 aus S-(+)-Phenyl-alkansäuren.

Verbindungen aus S-(+)-Phenyl-alkansäuren und α-Hydroxy-alkansäuren sind bereits bekannt. Es handelt sich dabei allerdings nicht um die speziellen erfindungsgemäßen Komplexe, sondern um Salze. Zum Stande der Tecknik ist insoweit zu verweisen auf die DE 38 36 863 A1.

Die Offenlegungsschrift DE 38 36 863 A1 (1990) beschreibt das γ-Butyrolacton, das wasserunlösliche, nichtsteroidiale Antirheumatika u.a. auch (R,S)-Ibuprofen gut lösen kann. Als Lösungen sind mit Wasser und anderen Flüssigkeiten mischbare Ansätze genannt, die sich auch beliebig verdünnen lassen. Das γ-Butyrolacton wird dabei im Organismus in die 4-Hydroxybuttersäure säure geöffnet und in $CO_2$ und Wasser metabolisiert. Im Beispiel 1, wird eine pharmazeutische Formulierung in Form einer Creme, einer Salbe oder eines Gels, offenbart, welche u.a. 1 ml Milchsäure enthält. Der Zusatz von R,S oder D,L-Milchsäure ist in einem nichtstöchiometrischen Verhältnis (Molbasis) in der (R,S)-Ibuprofen Formulierung angegeben.

Eine Aufgabe der vorliegenden Erfindung ist es, neue Stoffe auf der Basis von S-(+)-Phenyl-alkansäuren und α-Hydroxy-alkansäuren zu schaffen und deren vorteilhafte Verwendung in pharmazeutischen Zubereitungen zu erarbeiten.

Diese Aufgabe wird erfindungsgemäß gelöst durch wasserstoffbrücken-gebundene Komplexe mit einer Stöchiometrie von 1:1 aus S-(+)-Phenyl-alkansäuren und α-Hydroxyalkansäuren, bei welchen die Komplex-Bindung auf Karboxylat-Karboxyl-Wechselwirkungen mit einem Proton-Switch der Form $R_1\text{-COOH}\ldots{}^{-}\text{OOC-}R_2 \rightleftharpoons R_1\text{-COO}^{-}\ldots\text{HOOC-}R_2$ beruht, wobei $R_1$-COOH die S-(+)-Phenyl-alkansäuren und $R_2$-COOH die α-Hydroxyalkansäuren bedeuten, und wobei die pKa-Werte bezüglich der Karboxylgruppe der S-(+)-Phenyl-alkansäuren im Bereich von 3,5 - 3,9 und die pKa-Werte bezüglich der Karboxylgruppe der α-Hydroxyelkansäuren im Bereich von 1,8 - 2,9 liegen.

Vorzugsweise haben die α-Hydroxy-alkansäuren die folgende allgemeine Formel

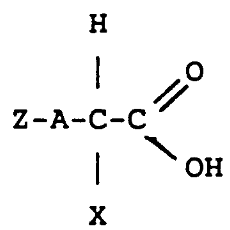

mit

X = OH
Z = H, OH, $NH_2$
A = eine Bindung oder eine Alkylenkette, welche 1 - 10 Kohlenstoffatome enthält oder bei Bedarf eine Aminogruppe oder 1 - 6 Hydroxylgruppen

Vorzugsweise liegen die die pKa-Werte bezüglich der Karboxylgruppe der α-Hydroxyalkansäuren im Bereich von 1,9 - 2,5.

Vorzugsweise enthalten die Komplexe die Enantiomeren der Milchsäure.

Vorzugsweise sollen hier als S-(+)-Phenyl-alkansäuren auch S-(+)-Ibuprofen oder S-(+)-Naproxen verstanden und verwendet werden.

Vorzugsweise sollen hier als S-(+)-Phenyl-alkansäuren auch die nachfolgend beschriebenen Substanzen verstanden und verwendet werden. Diesen Verbindungen ist die untenstehende Strukturformel gemeinsam,

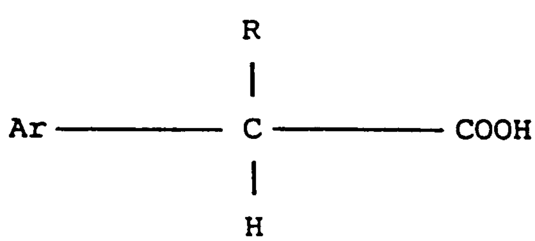

in welcher R ein Alkylrest ist, Ar vorzugsweise eine monozyklische, polyzyklische oder ortho-kondensierte polyzyklische aromatische Gruppe mit bis zu 12 Kohlenstoffatomen in dem aromatischen System, z.B. Phenyl, Diphenyl und Naphtyl. Die Substituenten dieser aromatischen Gruppen enthalten ein oder mehrere Halogenatome, $C_1$-$C_4$-Alkyle, Benzyl, Hydroxy, $C_1$-$C_2$-Alkoxy, Phenoxy und Benzoyl-Gruppen. Beispiele dieser substituierten Aryle sind: 4-Isobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl.

Vorzugsweise bestehen die Komplexe aus S-(+)-Phenyl-alkansäuren, vorzugsweise aus S-(+)-Ibuprofen und $\alpha$-Hydroxy-alkansäuren, vorzugsweise der D-Form der $\alpha$-Hydroxy-alkansäuren.

Erfindungsgemäß werden die erfindungsgemäßen Komplexe durch folgende Verfahrensschritte hergestellt:

a) Zur Herstellung aus wäßrigem Medium (nur Wasser) oder schwach gepufferten wäßrigen Lösungen, die einen pH-Bereich umfassen zwischen pH 5.5-7.5 (20°C) bereitet man eine gepufferte, wäßrige Lösung, z.B. einen 0.01M-0.001 M-$K_2HPO_4$/$KH_2PO_4$-Puffer pH 6.0-7.5 (20°C) vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird (normalerweise nach 20 Minuten) und alle S-(+)-Phenyl-alkansäure in Lösung gegangen ist;
c) anschließend wird der pH der Lösung auf pH 5.5-6.0 unter Zugabe von verdünnter Phosphorsäure ($H_3PO_4$) eingestellt (20°C) und danach wird die äquivalente (entsprechende) Menge der $\alpha$-Hydroxyalkansäure unter stetem Rühren eingetragen;
d) die Komplexbildung ist nach 20 Minuten beendet, worauf nach Abkühlen auf 0-4°C die Komplexe kristallin anfallen und über eine Glasnutsche oder einen Glasfilter (1G4) von der Mutterlauge abgetrennt werden können;
e) alternativ zu Verfahrensschritt d) kann die klare Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert und aus Wasser/Ethanol ($^{70}/_{30}$ $^{V}/_{V}$) oder aus Ethylacetat (100 %) umkristallisiert werden kann.

Bei den erfindungsgemäßen Stoffen handelt es sich nicht um eine Salzbildung zwischen einer sauren Gruppierung (Karboxylgruppe des Ibuprofens) und einem basischen Rest der $\alpha$-Hydroxy-alkansäure, sondern, wie die Röntgenstrukturanalyse und die FT-IR-Spektren zeigen, um Karboxylat-Karboxyl-Wechselwirkungen, wobei sich beide Karboxyl-Reste der $\alpha$-Hydroxy-alkansäure und z.B. des Ibuprofens, ein Proton teilen. D.h. der Komplex wird entsprechend der Röntgenstrukturanalyse durch eine Wasserstoffbrücke gebildet, ohne daß eine Beteiligung einer basischen Gruppe zu beobachten ist.

Die erfindungsgemäßen Komplexe können vorteilhaft in pharmazeutischen Zubereitungen verwendet werden, enthaltend einen oder mehrere Komplexe und gegebenenfalls wahlweise zusätzlich physiologisch verträgliche gebräuchliche Streckungsmittel oder Träger.

Insbesondere vorteilhaft ist eine pharmazeutische Zubereitung auf der Grundlage von Phenyl-alkansäuren mit antiinflammatorischer, antipyretischer, antimikrobieller und analgetischer Wirkung, enthaltend einen Wirkstoffkomplex aus einer Phenyl-alkansäure und einer $\alpha$-Hydroxy-alkansäure und ggf. zusätzlich übliche und physiologisch verträgliche Hilfsstoffe, bei der der Wirkstoffkomplex aus S-(+)-Phenyl-alkansäuren und $\alpha$-Hydroxy-alkansäuren besteht.

Insbesondere vorteilhaft ist eine pharmazeutische Zubereitung auf der Grundlage von Ibuprofen oder Naproxen mit antiinflammatorischer, antipyretischer, antimikrobieller und analgetischer Wirkung, enthaltend einen Wirkstoffkomplex aus einer Ibuprofen oder Naproxen und $\alpha$-Hydroxy-alkansäuren und ggf. zusätzlich übliche und physiologisch verträgliche Hilfsstoffe, bei der der Wirkstoffkomplex aus S-(+)-Ibuprofen oder S-(+)-Naproxen und einer $\alpha$-Hydroxy-alkansäure besteht und einen Gewichtsanteil 0,1 bis 90% (w/w) der Zusammensetzung ausmacht.

Insbesondere vorteilhaft ist eine pharmazeutische Zusammensetzung, die 50 bis 800 mg, vorzugsweise 100 bis 600 mg, insbesondere 100 bis 300 mg S-(+)-Ibuprofen bzw. S-(+)-Naproxen enthält.

Insbesondere vorteilhaft ist eine pharmazeutische Zubereitung, bei der die geeignete Dosis für die orale oder die parenterale Verabreichung im Bereich von 50 - 1200 mg pro Tag, normalerweise zwischen 100 und 800 mg pro Tag, vorzugsweise zwischen 200 und 600 mg S-(+)-Ibuprofen pro Tag liegt und daß die geeignete Dosis bei einer topischen Anwendung des Komplexes im Bereich von 10 - 200 mg pro Tag liegt.

Im folgenden wird die "pharmazeutisch aktive Verbindung" im weiteren Sinne als ein Komplex bezeichnet. In der medizinischen Anwendung kann diese pharmazeutisch aktive Verbindung oral, rektal, parenteral oder topisch angewendet werden, insbesondere jedoch oral oder topisch. Daher kann die therapeutische Zusammensetzung der vorliegenden Erfindung jede an sich bekannte pharmazeutische Zubereitung für orale, rektale, parenterale oder topische Anwendungen sein. Pharmazeutische gebräuchliche Träger, welche in solchen pharmazeutischen Zusammensetzungen angewendet werden, sind in der Pharmazie häufig beschrieben. Die Zusammensetzung dieser Erfindung kann 0,1 - 90% (w/w) der aktiven Verbindung entsprechen. Den Zusammensetzungen entsprechen normale, einheitliche Dosierungsformen. Diese Dosierungsformen enthalten 50 - 800 mg, vorzugsweise 100 - 600 mg oder 100 - 300 mg S-(+)-Ibuprofen.

Es werden orale Verabreichungsformen gemäß dieser Erfindung bevorzugt, wie z. B. Tabletten, Kapseln, Sirup und wäßrige oder ölige Suspensionen. Tabletten können z. B. hergestellt werden durch Mischen der aktiven Verbindung mit inerten Streckungsmitteln wie z. B. Calciumphosphat in Gegenwart eines aufschließenden Hilfsstoffes z. B. Stärke, oder Schmiermittels, z. B. Magnesiumstearat mit anschließender Tablettierung im normalen Herstellungssinne. Die Tabletten können in Form einer Retardformulierung der aktiven Verbindung nach bekannten Methoden hergestellt werden. Falls wünschenswert, können solche Tabletten nach entsprechend bekannten Methoden hergestellt werden, die sich im Magen nicht zersetzen; zum Beispiel unter Zuhilfenahme von Cellulose, Acetat, Phthalat. Entsprechend können Kapseln, z. B. Weich- und Hartgelatine-Kapseln hergestellt werden, welche die pharmazeutisch aktive Verbindung allein oder in Gegenwart von zugegebenen Hilfsstoffen enthalten. Diese Kapseln können mit üblicher pharmazeutischer Technologie, mit oder ohne magenresistenter Umhüllung, hergestellt werden. Andere Zusammensetzungen für orale Verabreichung schließen wäßrige Lösungen, die die aktive pharmazeutische Verbindung enthalten, in Gegenwart eines nicht toxischen Suspendierungsmittels, z. B. Carboxymethylcellulose und ölige Suspensionen, welche die aktive pharmazeutische Verbindung in Gegenwart eines Pflanzenöls.

Für die topische Anwendung der aktiven pharmazeutischen Verbindung können entsprechend dieser Erfindung pharmazeutische Formulierungen eingesetzt werden. Die pharmazeutisch aktive Verbindung wird in diesem Falle in einer pharmazeutisch geeigneten Creme, Salbe oder einem Gel dispergiert. Eine geeignete Creme kann z. B. dadurch hergestellt werden, daß die aktiv pharmazeutische Verbindung in einem topischen Träger wie z. B. leichtflüssiges Paraffin in einem wäßrigen Medium unter Zuhilfenahme von oberflächenaktiven Stoffen (Detergentien) dispergiert werden. Eine Salbe kann z. B. durch Mischen der pharmazeutisch aktiven Verbindung mit einem topischen Träger, wie z. B. Mineralöl oder Paraffin oder Bienenwachs hergestellt werden. Eine gelartige Formulierung kann durch Mischen einer aktiven pharmazeutischen Verbindung mit einem topischen Träger, z. B. Carbomer BP, in Gegenwart von Wasser hergestellt werden. Topisch anwendbare Zusammensetzungen können u.a. aus einer Matrix bestehen, die in der Lage ist, die aktive pharmazeutische Verbindung so zu dispergieren, daß diese durch ihren engen Kontakt mit der Haut transdermal verabreicht wird. Eine geeignete transdermale Zusammensetzung kann u. a. durch Mischen der pharmazeutisch aktiven Verbindung mit einem topischen Träger, wie vorne beschrieben, zusammen mit einem möglichen transdermalen Beschleuniger, z. B. Dimethylsulfoxid oder Propylenglykol, hergestellt werden.

Pharmazeutische Formulierungen entsprechend dieser Erfindung, die geeignet sind für die rektale Anwendung sind u. a. Suppositorien auf der Basis von Polyethylenglykol oder Kakaobutter.

Pharmazeutische Formulierungen zur parenteralen Anwendung beinhalten bekannte pharmazeutische Formulierungen z. B. sterile Suspensionen oder sterile Lösungen in einem geeigneten Lösungsmittel.

In einigen speziellen pharmazeutischen Formulierungen scheint es sinnvoll zu sein, die pharmazeutischen aktiven Verbindungen in einer Größe von kleinen Partikeln zu haben, z. B. kolloidale Lösungen oder partikuläre Suspensionen in der Größenordnung von 0,1 - 1 μm (Kolloidmühle).

Wenn wünschenswert, können entsprechend dieser Erfindung auch Zusammensetzungen mit anderen kompatiblen pharmazeutischen Wirkstoffen hergestellt werden.

Diese Komplexe entsprechend der Erfindung haben antiinflammatorische, antipyretische und interessante antimikrobielle Eigenschaften sowie analgetische Effekte. Diese Komplexe haben u. a. den Vorteil, daß sie nach oraler Verabreichung nach kürzerer Zeit wesentlich höhere Plasmaspiegel von S-(+)-Ibuprofen liefern als S-(+)-Ibuprofen in Form der freien Säure. Daher sind diese Komplexe in der Anwendung besonders wichtig zur Behandlung des akuten Schmerzes, wobei eine schnelle Anflutung mit sofortiger Schmerzbefreiung erzielt werden kann. Die Behandlung von Entzündungen und Schmerzen ist insbesondere wichtig bei Rheumapatienten, mit Indikationen wie primär chronische Polyarthritis, Arthritiden rheumatischen Ursprungs, Gelenkrheumatismus und Muskelrheumatismus mit ihren entsprechenden Schweregraden. Diese neuen Komplexe sind insbesondere wertvoll zur Schmerzentlastung, wie z. B. Kopfschmerzen, Dysmenorrhoe, postoperativer Schmerz, post-partum Schmerz und Schmerzen, die im Zusammenhang mit Grippe und Erkältungskrankheiten stehen.

Demgemäß beschreibt die Erfindung insbesondere einen anderen Aspekt zur Behandlung von Schmerz oder entzündlichem Fieber nach Verabreichung einer therapeutisch effektiven Dosis dieses Komplexes. Obwohl die genaue Dosis der pharmazeutisch aktiven Verbindung von einer Vielzahl von Parametern abhängig ist, z. B. Alter des Patienten, Zustand des Patienten, Anamnese und Compliance, liegt eine geeignete Dosis für sowohl orale als auch parenterale Verabreichungen von S-(+)-Ibuprofen-Komplex im Bereich von 50 - 1200 mg pro Tag, normalerweise zwischen 100 und 800 mg pro Tag, vorzugsweise zwischen 200 und 600 mg S-(+)-Ibuprofen pro Tag nach einmaliger oder mehrmaliger Verabreichung.

Bei einer topischen Anwendung dieses Komplexes liegt die entsprechende Dosis im Bereich von 10 - 200 mg pro Tag, im allgemeinen liegt die Dosis bei 20 - 100 mg pro Tag, je nach ärztlicher Anordnung.

Vorteilhaft können auch erfindungsgemäß die erfindungsgemäßen Komplexe in pharmazeutischen Zubereitungen verwendet werden, wie sie in der deutschen Anmeldung DE 40 15 794.6 beschrieben sind. Solche isotropen Lösungen können hergestellt werden durch die folgenden Verfahrensschritte:

a.) Erwärmen des Trägers unter Rühren bis oberhalb des Schmelzpunktes, bis eine isotrope, transparente Flüssigkeit vorliegt;

b.) Messung der elektrischen Leitfähigkeit und der Viskosität bei der Temperatur des Schmelzpunktes, um das Vorliegen einer isotropen, transparenten Flüssigkeit zu gewährleisten;

c.) Bestimmung des refraktiven Index;

d.) Einstellen der gewünschten Konzentration des pharmazeutischen Wirkstoffs unter Beachtung des Molenbruches, der bei 37° C zwischen 0,001 und 0,67 liegen muß ;

e.) Eintragen des pharmazeutischen Wirkstoffes unter stetigem Rühren in das Lösungsmittel;

f.) Rühren des Ansatzes, bis der pharmazeutische Wirkstoff gelöst und eine transparente Lösung entstanden ist;

g.) Messung des differentiellen refraktiven Index-Inrementes $[(\Delta n/\Delta c)_{T/P=konstant}]$ zur Bestimmung der monomolekularen Lösung und/oder

h.) Kontrolle der nativen Konformation und der Monomolekularität des pharmazeutischen Wirkstoffes in der Lösung durch Messung des molaren Extinktionskoeffizienten im UV-Bereich und durch Aufnahme des Absorptionsspektrums sowie Feststellen der chiralen Konfiguration durch Messung im Polarimeter und/oder

i.) Messung der Trübung, um eine homogene Lösung sicherzustellen und/oder

k.) Messung der spezifischen Leitfähigkeit $[(\Omega)_{T,V=konstant}]$ zur Kontrolle der ionalen Konzentration in der isotropen Lösung;

l.) Abkühlen der klaren Lösung und Herstellung einer galenischen Formulierung ;

m.) weiteres Abkühlen der Lösung auf Raumtemperatur, bis die Lösung erstarrt ist.

Die Erfindung wird im folgenden am Beispiel des Reaktionspartners Milchsäure noch näher erläutert:

(R,S)-Milchsäure ist bei Raumtemperatur eine Flüssigkeit (Fp:16,8°C), während die enantiomeren Formen, D-(-) und L-(+)-Milchsäure dagegen feste Substanzen sind (Fp:53 - 54°C). Die Enantiomeren der Milchsäure sind in Wasser, Ethanol und Ether leicht löslich und nicht hygroskopisch, wie die D,L-Milchsäure. Es wurde nunmehr gefunden, daß sowohl D-(-) als auch L-(+)-Milchsäure mit S-(+)-Ibuprofen einen 1:1 Molekülkomplex, sowohl im Festen als auch im Flüssigen (wäßrige Lösung) bildet. Der Komplex wird hergestellt, indem man eine wäßrige Lösung von D-(-) oder L-(+)-Milchsäure bereitet und sie mit der entsprechenden, stöchiometrischen Menge, Molverhältnis 1:1 D-(-) oder L-(+)-Milchsäure und S-(+)-Ibuprofen bei 35°C erhitzt, umrührt, und auf 20°C wieder abkühlt. Bei weiterer Abkühlung auf 0 - 4°C, erscheinen plättchenartige Kristalle, die nach Filtration und Trocknen bei 20°C unter Vakuum in der Trockenpistole einen Schmelzpunkt von 63 - 65°C haben. Die optimale Drehung dieser Präparationen beläuft sich wie folgt
$[\alpha]^{20}_{550}$ + 27°C (= 1,5, $CHCl_3$), für L-(+)-Milchsäure x S-(+)-Ibuprofen Komplex;
$[\alpha]^{20}_{550}$ + 2,5°C (=2,5 in $H_2O$), für D-(-)-Milchsäure x S-(+)-Ibuprofen.

Bei Umkristallisation des L-(+)-Milchsäure S-(+)-Ibuprofen Komplexes aus wasserfreiem Ethanol ergeben sich andere (polymorph) kristalline Formen, die nach Pulveraufnahmen (Guinier-Verfahren) auch andere Zelldimensionen zeigen als diejenigen Kristalle, welche aus wäßrigen bzw. 50 % (v/v) wäßrig-ethanolischen Lösungen erhalten werden können. Die letztgenannten kristallinen Formen (aus wäßrig-ethanolischer Lösung) haben einen Schmelzpunkt von 58 - 60°C und enthalten nach gravimetrischen und DSC-Messungen ein Molekül Wasser als Hydrat, welches im Kristallgitter eingebaut ist. Andere polymorphe Formen wurden erhalten, wenn aus Methanol/Ethylacetat 50 % (v/v) kristallisiert wurde.

Nach röntgenstrukturanalytischen Untersuchungen liegt auch hier ein wasserstoffbrücken-gebundener Komplex zwischen der Karboxylgruppe des S-(+)-Ibuprofen und der Enantiomeren Form der Milchsäure - auch hier die Karboxylgruppe - vor. Nach Literaturangaben und eigenen konduktimetrischen Messungen liegt der pKa der Milchsäure bei 3,55 - 3,88, derjenige des Hydroxyls bei pKa ≈ 9,5, wobei bei diesem pH die Milchsäure nicht stabil ist. D.h. unter den gegebenen Herstellungsbedingungen und unter Einbeziehung der pKa-Werte des S-(+)-Ibuprofens und der Milchsäure handelt es sich hier ebenfalls wie bei den $\alpha$-Aminosäuren um Karboxyl-Karboxylat Wasserstoffbrücken-Bindungen, welche diesen Komplex bilden. - Allerdings haben weitere NMR- und kristallchemische Untersuchungen gezeigt, daß außerdem je nach Kristallisationsbedingungen, noch weitere Wasserstoffbrücken-Bindungen zwischen allen Hydroxylen der Milchsäure und den Karboxylgruppen des S-(+)-Ibuprofens auftreten. In beiden Fällen besteht auch hier keine salzartige oder Ionen-Paar-Bindung zwischen S-(+)-Ibuprofen und den Enantiomeren der Milchsäure. Kristalline Verbindungen zwischen dem S-(+)-Ibuprofen und dem Racemat der Milchsäure (D,L) konnten bei stöchiometrischem Umsatz in alkoholische Lösung ebenfalls erhalten werden. Diese Verbindungen bestehend aus S-(+)-Ibuprofen und (D,L)-Milchsäure haben einen Schmelzpunkt von 28°C; $[\alpha]^{20}_{550}$ = +51° (95%, EtoH), und zeigen die gleichen Strukturprinzipien wie vorne für die reinen Enantiomere der Milchsäure erläutert wurde.

Die pharmakokinetische und pharmakodynamische Verhaltensweise, aufgezeigt am Beispiel des S-(+)-Ibuprofen x L-(+)-Milchsäure Komplex, folgt dem von Komplexen bestehend aus S-(+)-Ibuprofen und $\alpha$-Aminosäuren oder Aminozuckern: rascher Wirkungseintritt mit einem $t_{max}$ von 20 Minuten, eine hohe AUC von 50 mg/ml x h im Verhältnis zur freien Säure von nur 40 mg/ml x h bei einem $t_{max}$ von 2 h, bei gleicher Wirkstoffmenge von 150 mg S-(+)-Ibuprofen. Aus diesen pharmakokinetischen Ergebnissen ist ersichtlich, daß die hier offenbarte pharmazeutische Zubereitung der freien Säure von S-(+)-Ibuprofen überlegen ist.

Beispiele

1. Herstellung von S-(+)-Ibuprofen-L-(+)-Milchsäure Komplexes 100 g L-(+)-Milchsäure werden in 250 ml Wasser unter Rühren bei 20°C gelöst (1,11 Mol). Unter stetem Rühren werden 226,8 g (1,1 Mol) S-(+)-Ibuprofen eingetragen wobei die Lösung langsam (innerhalb von 10 Minuten) auf 35 - 40°C erwärmt wird. Der pH der Lösung soll nicht über 6,0 steigen, wenn ein 0,001 M $Na_2HPO_4/NaH_2PO_4$ Puffer verwendet wird. Bei einfacher wäßriger Lösung (pH 5,5 - 6,0) ist darauf zu achten, daß der pH nicht unter 4,0 abfällt, so daß u.U. mit verdünnter NaOH wieder auf den ursprünglichen pH 5,5 - 6,0 zurücktitriert werden muß. Die Temperatur von 35 - 40° C ist strikt einzuhalten, damit keine Entmischung eintritt. Nach ungefähr 30 Minuten tritt eine klare Lösung ein, die auf 0 - 4°C abgekühlt wird, worauf sich Kristalle des Komplexes bilden.

Alternativ kann die klare Lösung auf 20°C abgekühlt und eingeengt werden auf ungefähr 120 ml, woraus sich ein feiner, weißer Niederschlag (amorph) bildet, der über einen 1G4-Glasfilter abfiltriert werden kann.

Das so hergestellte Material wird unter Vakuum in einer Trockenpistole bei 20 - 25°C über Nacht getrocknet. Ausbeute: 85 - 90 % der Theorie. Umkristallisiert werden kann aus 50 % (v/v) wäßriger-ethanolischer Lösung, oder Ethanol/Ethylacetat (50/50, v/v). Das Material, unkristalliert aus reinem organischen Lösungsmittel oder das getrocknete Pulver hat einen Fp: 63 - 65°C, $[\alpha]^{20}_{550}$ +27°C (=1,5, $CHCl_3$); Kristalle aus wäßrig ethanolischer Lösung (50 % v/v) haben einen Fp: 58 - 60°C (Zersetzung) und $[\alpha]^{20}_{550}$ + 21,0°C (c1,5, $CHCl_3$).

2. Herstellung von S-(+)-Ibuprofen-D-(-)-Milchsäure-Komplex. Es wird verfahren wie im Beispiel 1 angegeben. Schmelzpunkt des aus Wasser oder Ethanol/Ethylacelat (50 % v/v) erhaltenen Komplexes: Fp: 63°C; $[\alpha]^{20}_{550}$ + 2,5°C (= 2,5 in $H_2O$).

3. Herstellung von S-(+)-Ibuprofen-D,L-Milchsäure-Komplex. 50 g (D,L) Milchsäure (0,55 Mol) werden unter Rühren bei Raumtemperatur in Wasser (200 ml) gelöst. Unter stetem Rühren werden 113,4 g S-(+)-Ibuprofen eingetragen, wobei die Lösung auf 30°C innerhalb von 10 Minuten gebracht wird. Nach ungefähr einer halben Stunde tritt vollständige Lösung ein, so daß das Reaktionsgemisch auf 20°C abgekühlt werden kann, und nach Beispiel aufgearbeitet werden kann. Ausbeute: 90 % der Theorie. Fp = 28°C; $[\alpha]^{20}_{550}$ + 51°C (95 %, Ethanol).

Beispiel 4

Eine pharmazeutische Formulierung kann unter anderem bestehen aus:

| | |
|---|---|
| S-(+)-Ibuprofen, L-(+)-Milchsäure | 215,5 mg |
| Magnesiumstearat, Pulver NF | 5,0 mg |
| Poridoul USP | 20,0 mg |
| Hydroxypropyl Methylcellulose USP 6 CPS | 4,0 mg |
| Titandioxid USP | 1,5 mg |
| Tah USP, gereinigt | 0,5 mg |
| Hydroxypropylcellulose LF, NFL 0,3 % $SiO_2$ | 4,0 mg |

Beispiel 5

| | |
|---|---|
| S-(+)-Ibuprofen, L-(+)-Milchsäure | 307,0 mg |
| Magnesiumstearat, Pulver NF | 10,0 mg |
| Hydroxypropyl Methylcellulose USP 6 CPS | 5,0 mg |
| Tah, USP | 1,0 mg |
| Hydroxypropylcellulose LF, NFL 0,3 % | 5,0 mg |
| Povidon USP | 10,0 mg |
| Titandioxyd | 2,0 mg |

Beispiel 6

Eine pharmazeutische Formulierung für Injektionszwecke kann unter anderem bestehen aus:

| | |
|---|---|
| S-(+)-Ibuprofen, L-(+)-Milchsäure Komplex Entspricht 20,0 mg S-(+)-Ibuprofen | 28,7 mg |
| Mannit | 20,0 mg |
| Wasser für Injektionszwecke aufgefüllt auf | 1,0 ml |

Beispiel 7

Die Komplexe bestehend aus S-(+)-Ibuprofen und L-(+)- bzw. D-(-)-Milchsäure im Molverhältnis 1:1, können auch aus Salzen der Enantiomeren der Milchsäure hergestellt werden.

Entsprechend folgender Vorschrift können diese Komplexe fast quantitativ und mit hoher optimaler Reinheit erhalten werden.

100 g L-(-)-Lithiumlaktat (1,04 Mol) werden in einer Mischung von 100 ml Ethanol (96 %, p.a.) und 100 ml Wasser (deionisiert) bei 20°C unter Rühren gelöst. Unter stetem Rühren werden 214,5 g (1,04 Mol) S-(+)-Ibuprofen langsam zugegeben, wobei darauf zu achten ist, daß der pH-Wert der Reaktionsmischung nicht über pH 5,5 steigt, so daß Zugabe von 0,01N HCl nötig wird. Die Lösung wird auf 30°C für 1 Stunde erwärmt, anschließend mit Ethylacetat versetzt (150 ml), wobei das Lithiumsalz anfällt und der Komplex in der Esterphase sich befindet, und anschließend eingeengt. Die weitere Verfahrensweise schließt sich dem Beispiel 1 an.

## Patentansprüche

**Patentansprüche für folgende Vertragsstaaten : AT, BE, CH, DE, DK, FR, GB, IT, LI, LU, NL, SE**

1. Wasserstoffbrücken-gebundene Komplexe mit einer Stöchiometrie von 1:1 aus S-(+)-Phenyl-alkansäuren und α-Hydroxyalkansäuren, bei welchen die Komplex-Bindung auf Karboxylat-Karboxyl-Wechselwirkungen mit einem Proton-Switch der Form $R_1$-COOH...$^{-}$OOC-$R_2$ $\rightleftharpoons$ $R_1$-COO$^{-}$...HOOC-$R_2$ beruht, wobei $R_1$-COOH die S-(+)-Phenyl-alkansäuren und $R_2$-COOH die α-Hydroxyalkansäuren bedeuten, und wobei die pKa-Werte bezüglich der Karboxylgruppe der S-(+)-Phenyl-alkansäuren im Bereich von 3,5 - 3,9 und die pKa-Werte bezüglich der Karboxylgruppe der α-Hydroxyalkansäuren im Bereich von 1,8 - 2,9 liegen.

2. Komplexe nach Anspruch 1,
dadurch gekennzeichnet,
daß die α-Hydroxy-alkansäuren die folgende allgemeine Formel haben:

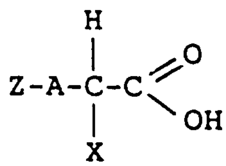

mit

X = OH
Z = H, OH, $NH_2$
A = eine Bindung oder eine Alkylenkette, welche 1 - 10 Kohlenstoffatome enthält oder bei Bedarf eine Aminogruppe oder 1 - 6 Hydroxylgruppen.

3. Komplexe nach einem oder mehreren der vorhergehenden Ansprüche,
dadurch gekennzeichnet,
daß die pKa-Werte bezüglich der Karboxylgruppe der α-Hydroxyalkansäuren im Bereich von 1,9 - 2,5 liegen.

**4.** Komplexe nach einem oder mehreren der vorhergehenden Ansprüche, dadurch gekennzeichnet, daß sie die Enantiomeren der Milchsäure enthalten.

**5.** Komplexe nach einem oder mehreren der vorhergehenden Ansprüche, dadurch gekennzeichnet, daß als S-(+)-Phenyl-alkansäuren S-(+)-Ibuprofen oder S-(+)-Naproxen verwendet werden.

**6.** Komplexe nach einem oder mehreren der vorhergehenden Ansprüche, dadurch gekennzeichnet, daß sie aus S-(+)-Ibuprofen und der D-Form der α-Hydroxy-alkansäuren bestehen.

**7.** Komplexe nach einem oder mehreren der vorhergehenden Ansprüche, dadurch gekennzeichnet, daß die S-(+)-Phenyl-alkansauren von der Form

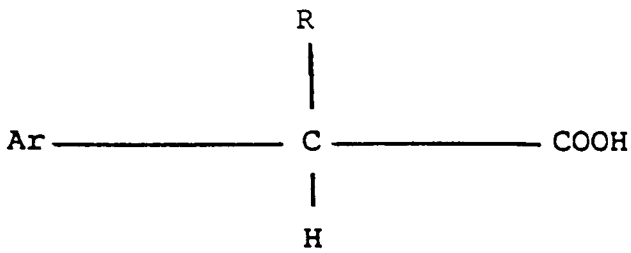

sind, in welcher R ein Alkylrest ist, Ar eine monozyklische, polyzyklische oder ortho-kondensierte polyzyklische aromatische Gruppe mit bis zu 12 Kohlenstoffatomen in dem aromatischen System, wie Phenyl, Diphenyl und Naphtyl, ist und wobei die Substituenten dieser aromatischen Gruppen ein oder mehrere Halogenatome, $C_1$-$C_4$-Alkyle, Benzyl, Hydroxy, $C_1$-$C_2$-Alkoxy, Phenoxy und Benzoyl-Gruppen enthalten.

**8.** Komplexe nach einem oder mehreren der vorhergehenden Patentansprüche, dadurch gekennzeichnet, daß die substituierten Aryle 4-Isobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl sind.

**9.** Verfahren zur Herstellung der Komplexe nach einem oder mehreren der Ansprüche gekennzeichnet durch folgende Verfahrensschritte:

a) Zur Herstellung aus wäßrigem Medium (nur Wasser) oder schwach gepufferten wäßrigen Lösungen, die einen pH-Bereich umfassen zwischen pH 5,5-7,5 (20°C) bereitet man eine gepufferte, wäßrige Lösung vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird und alle S-(+)-Phenyl-alkansäure in Lösung gegangen ist;
c) anschließend wird der pH der Lösung auf pH 5,5-6,0 unter Zugabe von verdünnter Phosphorsäure ($H_3PO_4$) eingestellt (20°C) und danach wird die äquivalente (entsprechende) Menge der α-Hydroxyalkansäure unter stetem Rühren eingetragen;
d) die Komplexbildung ist nach 20 Minuten beendet, worauf nach Abkühlen auf 0-4°C die Komplexe kristallin anfallen und über eine Glasnutsche oder einen Glasfilter (1G4) von der Mutterlauge abgetrennt werden können;
e) alternativ zu Verfahrensschritt d) kann die klare Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert und aus Wasser/Ethanol ($^{70}/_{30}$ $^{V}/_{V}$) oder aus Ethylacetat (100 %) umkristallisiert werden kann.

**10.** Pharmazeutische Zubereitung enthaltend einen oder mehrere Komplexe nach einem oder mehreren der vorhergehenden Ansprüche und wahlweise zusätzlich physiologisch verträgliche gebräuchliche Streckungsmittel oder Träger.

11. Pharmazeutische Zubereitung nach Anspruch 10 auf der Grundlage von Phenyl-alkansäuren mit antiinflammatorischer, antipyretischer, antimikrobieller und analgetischer Wirkung, enthaltend einen Wirkstoffkomplex aus einer Phenyl-alkansäure und einer α-Hydroxy-alkansäure und ggf. zusätzlich übliche und physiologisch verträgliche Hilfsstoffe,
dadurch gekennzeichnet,
daß der Wirkstoffkomplex aus S-(+)-Phenyl-alkansäuren und α-Hydroxy-alkansäuren besteht.

12. Pharmazeutische Zubereitung nach Anspruch 10 oder 11 auf der Grundlage von Ibuprofen oder Naproxen mit antiinflammatorischer, antipyretischer, antimikrobieller und analgetischer Wirkung, enthaltend einen Wirkstoffkomplex aus einer Ibuprofen oder Naproxen und α-Hydroxy-alkansäuren und ggf. zusätzlich übliche und physiologisch verträgliche Hilfsstoffe,
dadurch gekennzeichnet,
daß der Wirkstoffkomplex aus S-(+)-Ibuprofen oder S-(+)-Naproxen und einer α-Hydroxy-alkansäure besteht und einen Gewichtsanteil 0,1 bis 90% (w/w) der Zusammensetzung ausmacht.

13. Pharmazeutische Zusammensetzung nach einem oder mehreren der Ansprüche 10 bis 12,
dadurch gekennzeichnet,
daß sie 50 bis 800 mg, S-(+)-Ibuprofen oder S-(+)-Naproxen enthält.

14. Pharmazeutische Zusammensetzung nach einem oder mehreren der Ansprüche 10 bis 13,
bestehend aus einem oder mehreren Wirkstoffkomplexen und einem wasserlöslichen Träger, der im Bereich zwischen 20 und 80°C erstarrt und wasserlöslich ist,
dadurch gekennzeichnet,
daß sie eine isotrope Lösung ist, wobei

   a) der Wirkstoff in dem Träger monomolekular oder als Ion gelöst ist,
   b) der Wirkstoff in seiner nativen Konformation und/oder seiner biologisch aktiven chiralen (enantiomeren) Konformation vorliegt,
   c) der Wirkstoff einen Molenbruch von 0,001 bis 0,67 bei 37°C aufweist,
   d) der Träger bei Körpertemperatur schmelzflüssig, phaseneinheitlich und isotrop ist,
   e) die isotrope Lösung, bestehend aus Träger und Wirkstoff, bei Raumtemperatur erstarrt,
   f) die erstarrte Lösung kristallin oder nicht-kristallin ist oder sie den Wirkstoff kristallin enthält oder sie den Wirkstoff auskristallisieren kann,
   g) die monomolekulare oder ionische Lösung einen osmotischen Druck hat und eine molare Gefrierpunkterniedrigung bewirkt,
   h) der gelöste Wirkstoff innerhalb des Polymerelektrolyten einen temperaturabhängigen Diffusionskoeffizienten hat und eine temperaturabhängige spezifische Leitfähigkeit hat.

15. Pharmazeutische Zubereitung nach einem oder mehreren der Ansprüche 10 bis 14,
dadurch gekennzeichnet,
daS die geeignete Dosis für die orale oder die parenterale Verabreichung im Bereich von 50 - 1200 mg pro Tag, S-(+)-Ibuprofen pro Tag liegt und daß die geeignete Dosis bei einer topischen Anwendung des Komplexes im Bereich von 10 - 200 mg pro Tag liegt.

**Patentansprüche für folgenden Vertragsstaat : ES**

1. Verfahren zur Herstellung von wasserstoffbrücken-gebundenen Komplexen,
**dadurch gekennzeichnet**,
daß S-(+)-Phenyl-alkansäuren und α-Hydroxyalkansäuren so vermischt werden, daß Sie wasserstoffbrücken-gebundene Komplexe mit einer Stöchiometrie von 1:1 aus S-(+)-Phenylalkansäuren und α-Hydroxalkansäuren bilden, bei welchen die Komplex-Bindung auf Karboxylat-Karboxyl-Wechselwirkungen mit einem Proton-Switch der Form $R_1$-COOH...$^-$OOC-$R_2$ ⇋ $R_1$-COO$^-$...HOOC-$R_2$ beruht, wobei $R^1$-COOH die S-(+)-Phenyl-alkansäuren und $R_2$-COOH die α-Hydroxyalkansäuren bedeuten, und wobei die pKa-Werte bezüglich der Karboxylgruppe der S-(+)-Phenyl-alkansäuren im Bereich von 3,5 - 3,9 und die pKa-Werte bezüglich der Karboxylgruppe der α-Hydroxyalkansäuren im Bereich von 1,8 - 2,9 liegen.

2. Verfahren nach Anspruch 1,
dadurch gekennzeichnet,

daß die α-Hydroxy-alkansäuren die folgende allgemeine Formel haben:

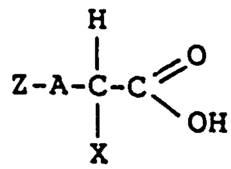

mit

X = OH

Z = H, OH, $NH_2$

A = eine Bindung oder eine Alkylenkette, welche 1 - 10 Kohlenstoffatome enthält oder bei Bedarf eine Aminogruppe oder 1 - 6 Hydroxylgruppen

**3.** Verfahren nach einem oder mehreren der vorhergehenden Ansprüche,
dadurch gekennzeichnet,
daß die pKa-Werte bezüglich der Karboxylgruppe der α-Hydroxyalkansäuren im Bereich von 1,9 - 2,5 liegen.

**4.** Verfahren nach einem oder mehreren der vorhergehenden Ansprüche,
dadurch gekennzeichnet,
daß sie die Enantiomeren der Milchsäure enthalten.

**5.** Verfahren nach einem oder mehreren der vorhergehenden Ansprüche,
dadurch gekennzeichnet,
daß als S-(+)-Phenyl-alkansäuren S-(+)-Ibuprofen oder S-(+)-Naproxen verwendet werden.

**6.** Verfahren nach einem oder mehreren der vorhergehenden Ansprüche,
dadurch gekennzeichnet,
daß sie aus S-(+)-Ibuprofen und der D-Form der α-Hydroxy-alkansäuren bestehen.

**7.** Verfahren nach einem oder mehreren der vorhergehenden Ansprüche,
dadurch gekennzeichnet,
daß die S-(+)-Phenyl-alkansäuren von der Form

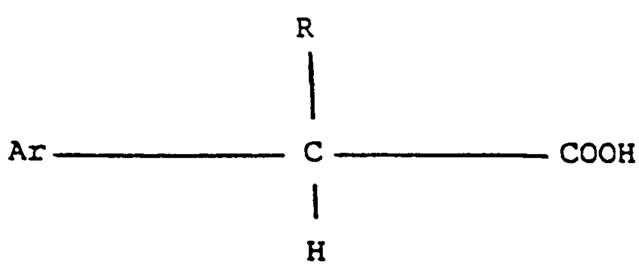

sind, in welcher R ein Alkylrest ist, Ar eine monozyklische, polyzyklische oder ortho-kondensierte polyzyklische aromatische Gruppe mit bis zu 12 Kohlenstoffatomen in dem aromatischen System, wie Phenyl, Diphenyl und Naphtyl ist und wobei die Substituenten dieser aromatischen Gruppen ein oder mehrere Halogenatome, $C_1$-$C_4$-Alkyle, Benzyl, Hydroxy, $C_1$-$C_2$-Alkoxy, Phenoxy und Benzoyl-Gruppen enthalten.

**8.** Verfahren nach einem oder mehreren der vorhergehenden Patentansprüche,
dadurch gekennzeichnet,
daß die substituierten Aryle 4-Isobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl sind.

**9.** Verfahren zur Herstellung der Komplexe nach einem oder mehreren der Ansprüche gekennzeichnet durch folgende Verfahrensschritte:

a) Zur Herstellung aus wäßrigem Medium (nur Wasser) oder schwach gepufferten wäßrigen Lösungen, die einen pH-Bereich umfassen zwischen pH 5,5-7,5 (20°C) bereitet man eine gepufferte, wäßrige Lösung vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird und alle S-(+)-Phenyl-alkansäure in Lösung gegangen ist;
c) anschließend wird der pH der Lösung auf pH 5,5-6,0 unter Zugabe von verdünnter Phosphorsäure ($H_3PO_4$) eingestellt (20°C) und danach wird die äquivalente (entsprechende) Menge der α-Hydroxyalkansäure unter stetem Rühren eingetragen;
d) die Komplexbildung ist nach 20 Minuten beendet, worauf nach Abkühlen auf 0-4°C die Komplexe kristallin anfallen und über eine Glasnutsche oder einen Glasfilter (1G4) von der Mutterlauge abgetrennt werden können;
e) alternativ zu Verfahrensschritt d) kann die klare Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert und aus Wasser/Ethanol ($^{70}/_{30}$ $^{V}/_{V}$) oder aus Ethylacetat (100 %) umkristallisiert werden kann.

**10.** Verfahren zur Herstellung einer eine oder mehrere der Komplexe nach einem oder mehreren der vorhergehenden Ansprüche enthaltenden pharmazeutischen Zubereitung,
**dadurch gekennzeichnet**,
daß eine oder mehrere der Komplexe nach einem oder mehreren der vorhergehenden Ansprüche mit physiologisch verträglichen Streckungsmitteln oder Trägern zur pharmazeutischen Zubereitung formuliert werden.

**11.** Verfahren nach Anspruch 10,
dadurch gekennzeichnet,
daß solche Komplexe gebildet werden, die eine antiinflammatorische, antipyretische, antimikrobielle und analgetische Wirkung aufweisen.

**12.** Verfahren nach einem oder mehreren der vorhergehenden Ansprüche,
dadurch gekennzeichnet,
daß der Wirkstoff aus S-(+)-Ibuprofen oder S-(+)-Naproxen und einer α-Hydroxy-alkansäure besteht und einen Gewichtsanteil von 0,1 bis 90 % (w/w) der Zusammensetzung ausmacht.

**13.** Verfahren nach einem oder mehreren der vorhergehenden Ansprüche,
dadurch gekennzeichnet,
daß 50 bis 800 mg S-(+)-Ibuprofen oder S-(+)-Naproxen verwendet werden.

**14.** Verfahren zur Herstellung einer pharmazeutischen Zubereitung, bestehend aus einem oder mehreren Komplexen als Wirkstoff und einem Träger, der im Bereich zwischen 20 und 80°C erstarrt und wasserlöslich ist, nach einem oder mehreren der vorhergehenden Ansprüche,
dadurch gekennzeichnet,
daß eine isotrope Lösung hergestellt wird, wobei

a) der Wirkstoff in dem Träger monomolekular oder als Ion gelöst ist,
b) der Wirkstoff in seiner nativen Konformation und/oder seiner biologisch aktiven chiralen (enantiomeren) Konformation vorliegt,
c) der Wirkstoff einen Molenbruch vor 0,001 bis 0,67 bei 37°C aufweist,
d) der Träger bei Körpertemperatur schmelzflüssig, phaseneinheitlich und isotrop ist,
e) die isotrope Lösung, bestehend aus Träger und Wirkstoff, bei Raumtemperatur erstarrt,
f) die erstarrte Lösung kristallin oder nicht-kristallin ist oder sie den Wirkstoff kristallin enthält oder sie den Wirkstoff auskristallisieren kann,
g) die monomolekulare oder ionische Lösung einen osmotischen Druck hat und eine molare Gefrierpunkterniedrigung bewirkt,
h) der gelöste Wirkstoff innerhalb des Polymerelektrolyten einen temperaturabhängigen Diffusionskoeffizienten hat und eine temperaturabhängige spezifische Leitfähigkeit hat.

**15.** Verfahren nach einem oder mehreren der vorhergehenden Ansprüche, dadurch gekennzeichnet, daß zur oralen oder parenteralen Verabreichung die pharazeutische Zubereitung so formuliert wird, daß eine Dosis pro Tag von 50 - 1200 mg S-(+)-Ibuprofen eingestellt wird, und daß für eine topische Anwendung die Dosis des Komplexes so eingestellt wird, daß sie im Bereich von 10 - 200 mg pro Tag liegt.

**Claims**

**Claims for the following Contracting States : AT, BE, CH, DE, DK, FR, GB, IT, LI, LU, NL, SE**

**1.** Hydrogen-bridge-bound complexes having a stoichiometry of 1:1 comprising S(+)-phenyl alkane acids and α-hydroxyalkane acids in which the complex bond is based on carboxylate-carboxyl interactions with a proton switch of the form $R_1$-COOH...$^-$OOC-$R_2$ ⇌ $R_1$-COO$^-$... HOOC-$R_2$ where $R_1$-COOH denotes the S(+)-phenyl alkane acids and $R_2$-COOH the α-hydroxyalkane acids and the pKa values relating to the carboxyl group of the S(+)-phenyl alkane acids lie in the range of 3.5 - 3.9 and the pKa values relating to the carboxyl group of the α-hydroxyalkane acids lie in the range of 1.8 - 2.9.

**2.** Complexes according to claim 1, characterized in that α-hydroxyalkane acids have the following general formula:

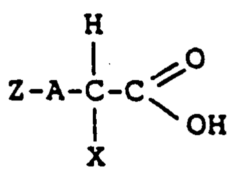

where

X = OH
Z = H, OH, $NH_2$
A = a bond or an alkylene chain which contains 1 - 10 carbon atoms and if required an amino group or 1 - 6 hydroxyl groups.

**3.** Complexes according to one or more of the preceding claims, characterized in that the pKa values relating to the carboxyl group of the α-hydroxyalkane acids lie in the range of 1.9 - 2.5.

**4.** Complexes according to one or more of the preceding claims, characterized in that they contain enentiomers of lactic acid.

**5.** Complexes according to one or more of the preceding claims, characterized in that as S(+)-phenyl alkane acids S(+)-ibuprofen or S(+)-naproxen are used.

**6.** Complexes according to one or more of the preceding claims, characterized in that they consist of S(+)-ibuprofen and the D-form of α-hydroxyalkane acids.

**7.** Complexes according to one or more of the preceding claims, characterized in that

the S(+)-phenyl alkane acids have a structure of the form

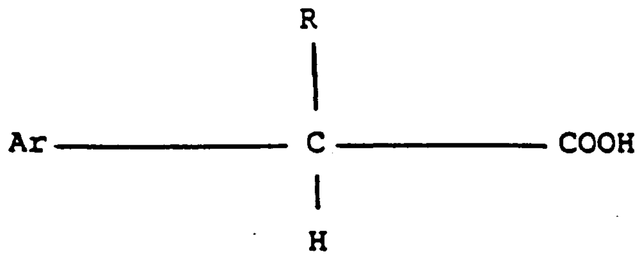

in which R is an alkyl residue, Ar is a monocyclic, polycyclic or ortho-condensed polycyclic aromatic group having up to twelve carbon atoms in the aromatic system, like phenyl, diphenyl, and naphthyl, and whereby the substituents of these aromatic groups comprise one or more halogen atoms, $C_1$-$C_4$-alkyls, benzyl, hydroxy, $C_1$-$C_2$ alkoxy, phenoxy and benzoyl groups.

**8.** Complexes according to one or more of the preceding claims,
characterized in
that the substituted aryls are: 4-isobutyl-phenyl, 3-phenoxy-phenyl, 2-fluoro-4-diphenyl, 4'-fluoro-4-diphenyl, 6-methoxy-2-naphthyl, 5-chloro-6-methoxy-2-naphthyl and 5-bromo-6-methoxy-naphthyl, 4-chloro-phenyl, 4-difluoromethoxy-phenyl, 6-hydroxy-2-naphthyl, and 5-bromo-6-hydroxy-2-naphthyl.

**9.** Method for the preparation of the complexes according to one or more of the preceding claims,
characterized by
the following method steps:

a) for the preparation from aqueous medium (only water) or weakly buffered aqueous solutions covering a pH range between pH 5.5 - 7.5 (20°C) a buffered aqueous solution is prepared and into it an equivalent amount S(+)-phenyl alkane acid is introduced with constant stirring;
b) the solution is heated with constant stirring to 40°C (water bath) until a clear transparent solution is obtained and all the S(+)-phenyl alkane acid has gone into solution;
c) thereafter the pH of the solution is adjusted to pH 5.5 - 6.0 by addition of diluted phosphoric acid ($H_3PO_4$) (20°C) and then the equivalent (corresponding) amount of the $\alpha$-amino acid is introduced with constant stirring;
d) the complex formation is terminated after 20 minutes, whereupon after cooling to 0 - 4°C the complexes precipitate in crystalline form and can be separated from the mother liquor via a sintered glass funnel or glass filter (1G4);
e) alternatively to method step d) the clear solution can be reduced in a rotary evaporator (water bath temperature 25 - 30°C) in the water jet vacuum to half the volume, whereupon a colourless (amorphous)deposit forms which is filtered off via a 1G4 glass filter and can be recrystallized from water/ethanol (70/30, v/v) or from ethyl acetate (100%).

**10.** Pharmaceutical preparation containing one or more complexes according to one or more of the preceding claims and optionally additionally physiologically compatible extenders or carriers.

**11.** Pharmaceutical preparation according to claim 10 on the basis of phenyl alkane acids having anti-inflammatory, antipyretic, antimicrobial and analgesic effect, containing an active substance complex comprising a phenyl alkane acid and an $\alpha$-hydroxalkane acid and possibly additionally usual physiologically compatible auxiliary substances,
characterized in
that the active substance complex consists of S(+)-phenyl alkane acids and basic $\alpha$-hydroxyalkane acids.

**12.** Pharmaceutical preparation according to claim 10 or 11 on the basis of ibuprofen or naproxen with anti-inflammatory, antipyretic, antimicrobial and analgesic effect, containing an active substance complex comprising an ibuprofen or naproxen and $\alpha$-hydroxyalkane acids and possibly additionally usual physiologically compatible auxiliary substances,
characterized in
that the active substance complex consists of S(+)-ibuprofen or (S-)-naproxen and an $\alpha$-hydroxyalkane acid and represents an amount by weight of 0.1 to 90% (w/w) of the composition.

**13.** Pharmaceutical preparation according to one or more of claims 10 to 12, characterized in that it contains 50 to 800 mg S(+)-ibuprofen or S(+)-naproxen.

**14.** Pharmaceutical preparation according to one or more of claims 10 to 13, consisting of one or more active substance complexes and a water-soluble carrier which solidifies in the range between 20 and 80°C and is water-soluble, characterized in that it is an isotropic solution, wherein

a) the active substance is dissolved in the carrier in monomolecular form or as ion,
b) the active substance is present in its native conformation and/or its biologically active chiral (enantiomeric) conformation,
c) the active substance has a molar fraction of 0.001 to 0.67 at 37°C,
d) the carrier is molten, phase-uniform and isotropic at body temperature,
e) the isotropic solution, consisting of carrier and active substance, solidifies at room temperature,
f) the solidified solution is crystalline or non-crystalline and contains the active substance in crystalline form or can crystallize the active substance out,
g) the monomolecular or ionic solution has an osmotic pressure and effects a molar freezing point reduction,
h) the dissolved active substance within the polymer electrolyte has a temperature-dependent diffusion coefficient and a temperature-dependent specific conductivity.

**15.** Pharmaceutical preparation according to one or more of claims 10 to 14, characterized in that the suitable dose for oral or parenteral administration is in the range of 50 - 1200 mg S(+)-ibuprofen daily and that the suitable doses for a topical administration of the complex lies in the range of 10 - 200 mg daily.

**Claims for the following Contracting State : ES**

**1.** A process for preparing hydrogen-bridge-bound complexes, characterized in that S(+)-phenyl alkane acids and α-hydroxyalkane acids are mixed in such a way that hydrogen-bridge-bound complexes having a stoichiometry of 1:1 comprising S(+)-phenyl alkane acids and α-hydroxyalkane acids are formed in which the complex bond is based on carboxylate-carboxyl interactions with a proton switch of the form $R_1$-COOH...$^-$OOC-$R_2 \rightleftharpoons R_1$-COO$^-$ ... HOOC-$R_2$ where $R_1$-COOH denotes the S(+)-phenyl alkane acids and $R_2$-COOH the α-hydroxyalkane acids and the pKa values relating to the carboxyl group of the S(+)-phenyl alkane acids lie in the range of 3.5 - 3.9 and the pKa values relating to the carboxyl group of the α-hydroxyalkane acids lie in the range of 1.8 - 2.9.

**2.** Process according to claim 1, characterized in that α-hydroxyalkane acids have the following general formula:

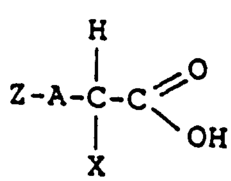

where

X = OH
Z = H, OH, $NH_2$
A = a bond or an alkylene chain which contains 1 - 10 carbon atoms and if required an amino group or 1 - 6 hydroxyl groups.

**3.** Process according to one or more of the preceding claims, characterized in that the pKa values relating to the carboxyl group of the α-hydroxyalkane acids lie in the range of 1.9 - 2.5.

**4.** Process according to one or more of the preceding claims, characterized in that they contain enentiomers of lactic acid.

**5.** Process according to one or more of the preceding claims, characterized in that as S(+)-phenyl alkane acids S(+)-ibuprofen or S(+)-naproxen are used.

**6.** Process according to one or more of the preceding claims, characterized in that they consist of S(+)-ibuprofen and the D-form of α-hydroxyalkane acids.

**7.** Process according to one or more of the preceding claims, characterized in that the S(+)-phenyl alkane acids have a structure of the form

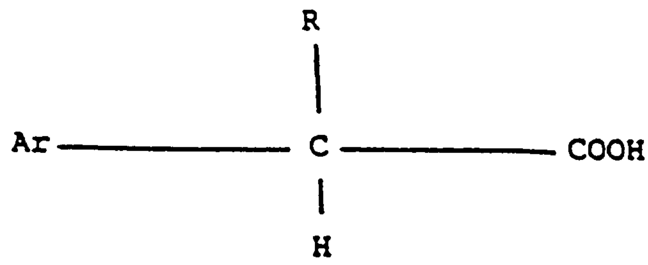

in which R is an alkyl residue, Ar is a monocyclic, polycyclic or ortho-condensed polycyclic aromatic group having up to twelve carbon atoms in the aromatic system, like phenyl, diphenyl, and naphthyl, and whereby the substituents of these aromatic groups comprise one or more halogen atoms, $C_1$-$C_4$-alkyls, benzyl, hydroxy, $C_1$-$C_2$ alkoxy, phenoxy and benzoyl groups.

**8.** Process according to one or more of the preceding claims, characterized in that the substituted aryls are: 4-isobutyl-phenyl, 3-phenoxy-phenyl, 2-fluoro-4-diphenyl, 4'-fluoro-4-diphenyl, 6-methoxy-2-naphthyl, 5-chloro-6-methoxy-2-naphthyl and 5-bromo-6-methoxy-naphthyl, 4-chloro-phenyl, 4-difluoromethoxy-phenyl, 6-hydroxy-2-naphthyl, and 5-bromo-6-hydroxy-2-naphthyl.

**9.** Process for the preparation of the complexes according to one or more of the preceding claims, characterized by the following process steps:

a) for the preparation from aqueous medium (only water) or weakly buffered aqueous solutions covering a pH range between pH 5.5 - 7.5 (20°C) a buffered aqueous solution is prepared and into it an equivalent amount S(+)-phenyl alkane acid is introduced with constant stirring;
b) the solution is heated with constant stirring to 40°C (water bath) until a clear transparent solution is obtained and all the S(+)-phenyl alkane acid has gone into solution;
c) thereafter the pH of the solution is adjusted to pH 5.5 - 6.0 by addition of diluted phosphoric acid ($H_3PO_4$) (20°C) and then the equivalent (corresponding) amount of the α-amino acid is introduced with constant stirring;
d) the complex formation is terminated after 20 minutes, whereupon after cooling to 0 - 4°C the complexes precipitate in crystalline form and can be separated from the mother liquor via a sintered glass funnel or glass filter (1G4);
e) alternatively to method step d) the clear solution can be reduced in a rotary evaporator (water bath temperature 25 - 30°C) in the water jet vacuum to half the volume, whereupon a colourless (amorphous) deposit forms which is filtered off via a 1G4 glass filter and can be recrystallized from water/ethanol (70/30, v/v) or from ethyl acetate (100%).

**10.** Process for preparing a pharmaceutical preparation comprising one or more of the complexes according to one or more of the preceding claims, characterized in that one or more of said complexes according to one or more of the preceding claims are formulated together with physiologically compatible extenders or carriers to form the pharmaceutical preparation.

**11.** Process according to claim 10, characterized by forming complexes which show an anti-inflammatory, antipyretic, antimicrobial and analgesic effect.

**12.** Process according to one or more of the preceding claims, characterized in that the active substance consists of S(+)-ibuprofen or S(+)-naproxen and an α-hydroxyalkane acid and represents an amount by weight of 0.1 to 90% (w/w) of the composition.

**13.** Process according to one or more of the preceding claims, characterized in that 50 to 800 mg S(+)-ibuprofen or S(+)-naproxen are used.

**14.** Process for preparing a pharmaceutical preparation consisting of one or more complexes as active substance and a carrier which solidifies in the range between 20 and 80°C and is water-soluble according to one or more of the preceding claims, characterized in that an isotropic solution is prepared wherein

a) the active substance is dissolved in the carrier in monomolecular form or as ion,
b) the active substance is present in its native conformation and/or its biologically active chiral (enantiomeric) conformation,
c) the active substance has a molar fraction of 0.001 to 0.67 at 37°C,
d) the carrier is molten, phase-uniform and isotropic at body temperature,
e) the isotropic solution, consisting of carrier and active substance, solidifies at room temperature,
f) the solidified solution is crystalline or non-crystalline and contains the active substance in crystalline form or can crystallize the active substance out,
g) the monomolecular or ionic solution has an osmotic pressure and effects a molar freezing point reduction,
h) the dissolved active substance within the polymer electrolyte has a temperature-dependent diffusion coefficient and a temperature-dependent specific conductivity.

**15.** Process according to one ore more of the preceding claims, characterized in that for oral or parenteral administration, a pharmaceutical preparation is formulated so that a dose is in the range of 50 - 1200 mg S(+)-ibuprofen daily, and that for a topical administration the dosis of the complex is formulated so that it lies in the range of 10 to 200 mg daily.

**Revendications**

**Revendications pour les Etats contractants suivants : AT, BE, CH, DE, DK, FR, GB, IT, LI, LU, NL, SE**

**1.** Complexes d'acides S-(+)-phénylalcanoïques et d'acides α-hydroxyalcanoïques liés par des liaisons hydrogènes avec une stoechiométrie de 1:1, dans lesquels la liaison du complexe est basée sur les interactions carboxylate-carboxyle avec un échange de proton de la forme $R_1\text{-COOH}...^{-}\text{OOC-}R_2 \rightleftharpoons R_1\text{-COO}^{-}...\text{HOOC-}R_2$, dans lequel $R_1$-COOH représente l'acide S-(+)-phénylalcanoïque et $R_2$-COOH représente l'acide α-hydroxyalcanoïque et dans lequel les pKa du groupe carboxyle de l'acide S-(+)-phénylalcanoïque sont compris entre 3,5 et 3,9 et les pKa du groupe carboxyle de l'acide α-hydroxyalcanoïque sont compris entre 1,8 et 2,9.

**2.** Complexes selon la revendication 1, caractérisés en ce que, les acides α-hydroxyalcanoïques ont la formule générale suivante:

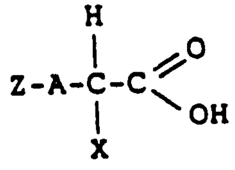

avec X = OH

Z = H, OH, $NH_2$
A = une liaison ou un chaîne alkyle comportant 1 à 10 atomes de carbone ou si besoin est, un groupe amino ou 1 à 6 groupes hydroxyles.

**3.** Complexes selon une ou plusieurs des revendications précédentes, caractérisés en ce que les pKa des groupes carboxyles des acides $\alpha$-hydroxyalcanoïques sont compris entre 1,9 et 2,5.

**4.** Complexes selon une ou plusieurs des revendications précédentes, caractérisés en ce qu'ils renferment les énantiomères de l'acide lactique.

**5.** Complexes selon une ou plusieurs des revendications précédentes, caractérisés en ce que le S-(+)-ibuprofène ou le S-(+)-naproxène sont utilisés à la place des acides S-(+)-phénylalcanoïques.

**6.** Complexes selon une ou plusieurs des revendications précédentes,
caractérisés en ce qu'ils se composent de S-(+)-ibuprofène et de la forme D de l'acide $\alpha$-hydroxyalcanoïque.

**7.** Complexes selon une ou plusieurs des revendications précédentes,
caractérisés en ce que les acides S-(+)-phénylalcanoïques sont de la forme

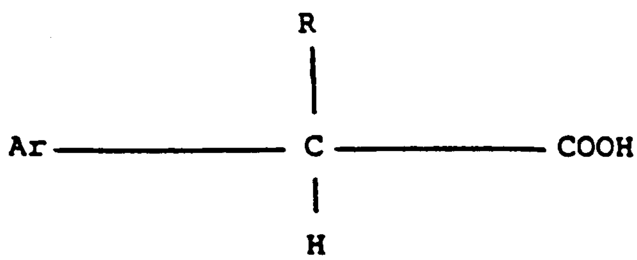

dans laquelle R est un résidu alkyle, Ar est un groupe aromatique monocyclique, polycyclique ou polycyclique orthocondensé ayant jusqu'à 12 atomes de carbone dans le système aromatique, tel que le phényle, le diphényle et le naphtyle et dans lesquels les substituants de ces groupes aromatiques renferment un ou plusieurs atomes d'halogènes, des groupes $C_1$-$C_4$-alkyle, benzyle, hydroxy, $C_1$-$C_2$-alcoxy, phénoxy et benzoyl.

**8.** Complexes selon une ou plusieurs des revendications précédentes,
caractérisés en ce que les aryles substitués sont le 4-isobutyl-phényle, le 3-phénoxy-phényl, le 2-fluoro-4-diphényle, le 4'-fluoro-4-diphényle, le 6-méthoxy-2-naphtyle, le 5-chloro-6-méthoxy-2-naphtyle et le 5-bromo-6-méthoxy-naphtyle, le 4-chloro-phényle, le 4-difluoro-méthoxy-phényle, le 6-hydroxy-2-naphtyle et le 5-bromo-6-hydroxy-2-naphtyle.

**9.** Procédé de fabrication de complexes selon une plusieurs des revendications
caractérisé par les étapes de fabrication suivantes:

a) pour la fabrication en milieu aqueux (seulement de l'eau) ou en solutions aqueuses faiblement tamponnées dont le pH est compris entre 5,5 et 7,5 (20°C), on prépare une solution aqueuse tamponnée dans laquelle on rajoute une quantité équivalente d'acide S-(+)-phénylalcanoïque avec une agitation constante ;

b) la solution est chauffée à 40°C (au bain-marie) avec une agitation constante jusqu'à l'obtention d'une solution claire, transparente et que tout l'acide S-(+)-phénylalcanoïque soit mis en solution;

c) ensuite le pH de la solution est ajusté à 5,5-6,0 par addition d'acide phosphorique dilué ($H_3PO_4$) (20°C) puis une quantité équivalente (correspondante) d'acide $\alpha$-hydroxyalcanoïque est rajoutée avec une agitation constante.

d) la formation du complexe est réalisée au bout de 20 minutes après quoi le complexe est précipité en cristaux par refroidissement à 0-4°C et peut être séparé de la solution mère sur un entonnoir filtre en verre ou sur un filtre en verre (1G4);

e) alternativement à l'étape de fabrication d) la solution claire peut être réduite à la moitié de son volume dans un évaporateur rotatif (température du bain-marie 25-30°C) sous une trompe à vide, ce qui fait apparaître un précipité incolore (amorphe) qui est filtré sur un filtre en verre 1G4 et peut être cristallisé dans un mélange eau/éthanol (70/30 V/V) ou dans de l'acétate d'éthyle (100%).

**10.** Composition pharmaceutique renfermant un ou plusieurs complexes selon une ou plusieurs des revendications précédentes et au choix un diluant ou un porteur courant physiologiquement compatibles.

**11.** Composition pharmaceutique selon la revendication 10 à base d'acides phényl-alcanoïques ayant une action anti-inflammatoire, antipyrétique, antimicrobienne et analgésique, comprenant un complexe actif d'un acide phényl-alcanoïque et d'un acide $\alpha$-hydroxyalcanoïque et le cas échéant un agent auxiliaire supplémentaire, courant et physiologiquement compatible,
caractérisée en ce que le complexe actif se compose des acides S-(+)-phénylalcanoïques et $\alpha$-hydroxyalcanoïques.

**12.** Composition pharmaceutique selon la revendication 10 ou 11 à base d'ibuprofène ou de naproxène ayant une action anti-inflammatoire, antipyrétique, antimicrobienne et analgésique, comprenant un complexe actif d'ibuprofène ou de naproxène et d'acides $\alpha$-hydroxyalcanoïques et le cas échéant un agent auxiliaire supplémentaire, courant et physiologiquement compatible,
caractérisée en ce que le complexe actif se compose de S-(+)-ibuprofène ou S-(+)-naproxène et d'un acide $\alpha$-hydroxyalcanoïque et représente 0,1 à 90% en poids (p/p) de la préparation.

**13.** Composition pharmaceutique selon une ou plusieurs des revendications 10 à 12,
caractérisée en ce que,
elle renferme 50 à 800 mg de S-(+)-ibuprofène ou de S-(+)-naproxène.

**14.** Composition pharmaceutique selon une ou plusieurs des revendications 10 à 13,
composée d'un ou plusieurs complexes actifs et d'un porteur soluble dans l'eau qui fige dans le domaine de température compris entre 20 et 80°C et qui est soluble dans l'eau,
caractérisée en ce que c'est une solution isotrope dans laquelle

a) l'agent actif est dissout dans le porteur sous forme d'ion ou sous forme monomoléculaire,

b) l'agent actif se présente sous sa conformation native et/ou sous sa conformation chirale biologiquement active (énantiomère),

c) l'agent actif présente une fraction de mole de 0,001 à 0,67 à 37°C,

d) le porteur est liquéfié, en une seule phase et isotrope à la température corporelle,

e) la solution isotrope, composée d'un porteur et d'un agent actif fige à la température ambiante,

f) la solution figée est cristalline ou non cristalline ou elle renferme l'agent actif sous forme cristalline ou elle peut cristalliser l'agent actif,

g) la solution monomoléculaire ou ionique a une tension osmotique et diminue le point de congélation molaire,

h) l'agent actif dissout dans le polymère électrolytique a un coefficient de diffusion dépendant de la température et une conductibilité spécifique dépendante de la température.

**15.** Composition pharmaceutique selon une ou plusieurs des revendications 10 à 14,
caractérisée en ce que la dose adaptée à l'administration orale ou parentérale est de l'ordre de 50 à 1200 mg par jour de S-(+)-ibuprofène et la dose adaptée à l'utilisation topique du complexe est de l'ordre de 10 à 200 mg par jour.

**Revendications pour l'Etat contractant suivant : ES**

**1.** Procédés de fabrication de complexes liés par des liaisons hydrogènes,
caractérisés en ce que,
les acides S-(+)-phénylalcanoïques et $\alpha$-hydroxyalcanoïques sont mélangés de telle façon qu'ils forment des complexes d'acide S-(+)-phénylalcanoïque et d'acide $\alpha$-hydroxyalcanoïque liés par des liaisons hydrogènes avec une

stoechiométrie de 1:1, dans lesquels la formation de complexe repose sur l'interaction carboxylate-carboxyle avec un échange de protons de la forme $R_1$-COOH...$^-$OOC-$R_2$ ⇋ $R_1$-COO$^-$...HOOC-$R_2$, dans lequel $R_1$-COOH représente l'acide S-(+)-phénylalcanoïque et $R_2$-COOH représente l'acide α-hydroxyalcanoïque et dans lequel les pKa du groupe carboxyle de l'acide S-(+)-phénylalcanoïque sont compris entre 3,5 et 3,9 et les pKa du groupe carboxyle de l'acide α-hydroxyalcanoïque sont compris entre 1,8 et 2,9.

2. Procédés selon la revendication 1,
caractérisés en ce que les acides α-hydroxyalcanoïques ont la formule générale suivante:

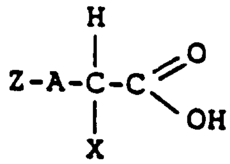

avec X = OH
Z = H, OH, $NH_2$
A = une liaison ou un chaîne alkyle comportant 1 à 10 atomes de carbone ou si besoin est, un groupe amino ou 1-6 groupes hydroxyles.

3. Procédés selon une ou plusieurs revendications précédentes,
caractérisés en ce que les pKa des groupes carboxyles des acides α-hydroxyalcanoïques sont compris entre 1,9 et 2,5.

4. Procédés selon une ou plusieurs des revendications précédentes,
caractérisés en ce qu'ils renferment les énantiomères de l'acide lactique.

5. Procédés selon une ou plusieurs des revendications précédentes,
caractérisés en ce que le S-(+)-ibuprofène ou le S-(+)-naproxène sont utilisés à la place des acides S-(+)-phénylalcanoïques.

6. Procédés selon une ou plusieurs des revendications précédentes,
caractérisés en ce qu'ils se composent de S-(+)-ibuprofène et de la forme D de l'acideα-hydroxyalcanoïque.

7. Procédé selon une ou plusieurs des revendications précédentes,
caractérisé en ce que,
les acides S-(+)-phénylalcanoïques sont de la forme

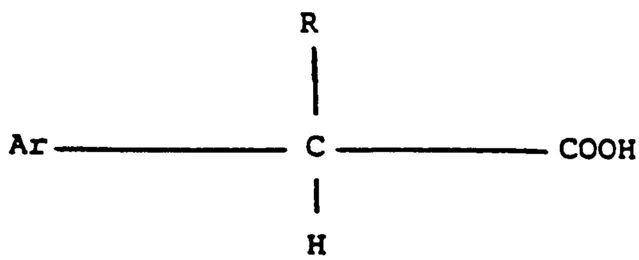

dans laquelle R est un résidu alkyle, Ar est un groupe aromatique monocyclique, polycyclique ou polycyclique orthocondensé ayant jusqu'à 12 atomes de carbone dans le système aromatique, tel que le phényle, le diphényle et le naphtyle et dans lesquels les substituants de ces groupes aromatiques renferment un ou plusieurs atomes d'halogènes, des groupes $C_1$-$C_4$-alkyle, benzyle, hydroxy, $C_1$-$C_2$-alcoxy, phénoxy et benzoyl.

8. Procédés selon une ou plusieurs des revendications précédentes,
caractérisés en ce que les aryles substitués sont le 4-isobutyl-phényle, le 3-phénoxy-phényl, le 2-fluoro-4-diphényle, le 4'-fluoro-4-diphényle, le 6-méthoxy-2-naphtyle, le 5-chloro-6-méthoxy-2-naphtyle et le 5-bromo-6-méthoxy-naphtyle, le 4-chloro-phényle, le 4-difluoro-méthoxy-phényle, le 6-hydroxy-2-naphtyle et le 5-bromo-6-hydroxy-2-naphtyle.

**9.** Procédés de fabrication de complexes selon une plusieurs des revendications caractérisés par les étapes de fabrication suivantes:

a) pour la fabrication en milieu aqueux (seulement de l'eau) ou en solutions aqueuses faiblement tamponnées dont le pH est compris entre 5,5 et 7,5 (20°C), on prépare une solution aqueuse tamponnée dans laquelle on rajoute une quantité équivalente d'acide S-(+)-phénylalcanoïque avec une agitation constante;

b) la solution est chauffée à 40°C (au bain-marie) avec une agitation constante jusqu'à l'obtention d'une solution claire, transparente et que tout l'acide S-(+)-phénylalcanoïque soit mis en solution;

c) ensuite le pH de la solution est ajusté à 5,5-6,0 par addition d'acide phosphorique dilué ($H_3PO_4$) (20°C) puis une quantité équivalente (correspondante) d'acide $\alpha$-hydroxyalcanoïque est rajoutée avec une agitation constante.

d) la formation du complexe est réalisée au bout de 20 minutes après quoi le complexe est précipité en cristaux par refroidissement à 0-4°C et peut être séparé de la solution mère sur un entonnoir filtre en verre ou sur un filtre en verre (1G4);

e) alternativement à l'étape de fabrication d) la solution claire peut être réduite à la moitié de son volume dans un évaporateur rotatif (température du bain-marie 25-30°C) sous une trompe à vide, ce qui fait apparaître un précipité incolore (amorphe) qui est filtré sur un filtre en verre 1G4 et peut être cristallisé dans un mélange eau/éthanol (70/30 V/V) ou dans de l'acétate d'éthyle (100%).

**10.** Procédés de fabrication d'une composition pharmaceutique renfermant un ou plusieurs complexes selon une ou plusieurs des revendications précédentes, caractérisés en ce qu'un ou plusieurs des complexes selon une ou plusieurs des revendications précédentes sont formulés pour la composition pharmaceutique avec un diluant ou des porteurs physiologiquement compatibles.

**11.** Procédé selon la revendication 10,
caractérisé en ce que de tels complexes sont élaborés de manière à leur conférer une action anti-inflammatoire, antipyrétique, antimicrobienne et analgésique.

**12.** Procédé selon une ou plusieurs des revendications précédentes,
caractérisé en ce que l'agent actif se compose de S-(+)-ibuprofène ou S-(+)-naproxène et d'un acide $\alpha$-hydroxyalcanoïque et représente 0,1 à 90% en poids (p/p) de la préparation.

**13.** Procédé selon une ou plusieurs des revendications précédentes,
caractérisé en ce qu'on utilise 50 à 800 mg de S-(+)-ibuprofène ou de S-(+)-naproxène.

**14.** Procédé de fabrication d'une composition pharmaceutique composée d'un ou plusieurs complexes actifs et d'un porteur soluble dans l'eau qui fige dans le domaine de température compris entre 20 et 80°C et qui est soluble dans l'eau,
caractérisé en ce qu'une solution isotrope est élaborée dans laquelle

a) l'agent actif est dissout dans le porteur sous forme monomoléculaire ou sous forme d'ion,

b) l'agent actif se présente sous sa conformation native et/ou sous sa conformation chirale biologiquement active (énantiomère),

c) l'agent actif présente une fraction de mole de 0,001 à 0,67 à 37°C,

d) l'agent porteur est liquéfié, en une seule phase et isotrope à la température corporelle,

e) la solution isotrope, composée d'un porteur et d'un agent actif se fige à la température ambiante,

f) la solution figée est cristalline ou non cristalline ou elle renferme l'agent actif sous forme cristalline ou elle peut cristalliser l'agent actif,

g) la solution monomoléculaire ou ionique a une tension osmotique et abaisse le point de congélation molaire,

h) l'agent actif dissout dans le polymère électrolytique a un coefficient de diffusion dépendant de la température et une conductibilité spécifique dépendante de la température.

**15.** Procédé selon une ou plusieurs des revendications précédentes,
caractérisé en ce que,
la composition pharmaceutique adaptée à l'administration orale ou parentérale est formulée de telle façon que la dose quotidienne est de l'ordre de 50 à1200 mg de S-(+)-ibuprofène et que la dose adaptée à l'utilisation topique du complexe est de l'ordre de 10 à 200 mg par jour.